# EUROPÄISCHE PATENTSCHRIFT

(11) **EP 1 318 129 B1**
(45) Veröffentlichungstag und Bekanntmachung des Hinweises auf die Patenterteilung: **22.11.2006**
(21) Anmeldenummer: 02027071.6
(22) Anmeldetag: 03.12.2002
(51) Int. Cl.: C07C 29/141, C07C 33/025

(54) **Verfahren zur selektiven Flüssigphasenhydrierung von Carbonylverbindungen zu den entsprechenden Alkoholen in Gegenwart eines Pt/ZnO-Katalysators**
Process for the selective liquid-phase hydrogenation of carbonyl compounds to the corresponding alcohols in the presence of a Pt/ZnO catalyst
Procédé pour l'hydrogénation sélective en phase liquide de composés carbonyles en alcools correspondants en présence d'un catalysateur Pt/ZnO

(30) Priorität: 07.12.2001 DE 10160141
(43) Veröffentlichungstag der Anmeldung: 11.06.2003
(73) Patentinhaber: BASF Aktiengesellschaft, 67056 Ludwigshafen (DE)
(72) Erfinder: Haake, Mathias, Dr., 68161 Mannheim (DE); Gerlach, Till, Dr., 67071 Ludwigshafen (DE); Funke, Frank, Dr., 68161 Mannheim (DE)

(56) Entgegenhaltungen:
- DE-A- 2 650 046
- US-A- 4 100 180
- US-A- 5 118 884
- US-B1- 6 294 696
- RECCHIA S. ET AL.: "Outstanding Performances of Magnesia-Supported Platinum-Tin Catalysts for Citral SElective Hydrogenation" JOURNAL OF CATALYSIS, Bd. 184, 1999, Seiten 1-4, XP002253596
- PAULOSE M.M. ET AL.: "Hydrogenation of Citral: II. Continuous and batch hydrogenation of Citral to geraniol-Nerol" JOURNAL OF THE OIL TECHNOLOGISTS' ASSOCIATION OF INDIA, 1974, Seiten 88-91, XP009007115

## Beschreibung

Die vorliegende Erfindung betrifft ein Verfahren zur selektiven Flüssigphasenhydrierung von α,β-ungesättigten Carbonylverbindungen zu den entsprechenden Alkoholen, insbesondere von Citral zu Geraniol/Nerol, oder von Citronellal zu Citronellol mit Wasserstoff in Gegenwart eines Pt/ZnO-Katalysators.

Aus dem Stand der Technik sind verschiedene Hydrierverfahren für α,β-ungesättigte Carbonylverbindungen bekannt. Es ist sehr schwierig hohe Selektivitäten der entsprechenden ungesättigten Alkohole zu erhalten. Bei der Hydrierung von Citral können neben der Aldehydgruppe auch die olefinischen Doppelbindungen hydriert werden, oder nur die zur Aldehydgruppe konjugierte Doppelbindung, so dass neben den ungesättigten Alkoholen Geraniol bzw. Nerol auch Nebenprodukte wie Citronellol oder Citronellal entstehen können.

U.S. 4,100,180 beschreibt ein Verfahren zur Hydrierung von ungesättigten Aldehyden zu ungesättigten Alkoholen in Gegenwart eines PtO/Zn/Fe-Katalysators. PtO-Pulver wird mit Zn und Fe dotiert (Suspensionskatalysator). Bei der Citralhydrierung werden bei 70 % Citralumsatz 3,2 % Citronellol erhalten. Im Reaktionsaustrag befinden sich bis zu 25 ppm Fe- und Zn-Verbindungen. Wird der Katalysator wiederverwendet müssen erneut geringe Mengen Fe- und Zn-Verbindung zugegeben werden. Die mit diesem Verfahren erzielten Citronellol-Selektivitäten sind unbefriedigend hoch und zusätzlich ist die Verwendung von PtO sehr teuer und macht das Verfahren unwirtschaftlich.

Neri et al. (J. Chem. Tech. Biotechnol. 60 (1994), 83-88) beschreiben ein selektives Hydrierverfahren von Citral an einem Pt-Sn-Katalysator auf Aktivkohle. Die Herstellung des Katalysators erfolgt durch gleichzeitige Imprägnierung der Aktivkohle mit Pt und Sn unter Verwendung von Chloriden. Die Citralhydrierung wird in Ethanol als Lösungsmittel durchgeführt. Durch die Zinnzugabe wird sowohl die Aktivität als auch die Selektivität erhöht. Dennoch sind die Citronellolselektivitäten größer als 5 %.

EP 071 787 offenbart Ruthenium/Kohle-Hydrierkatalysatoren, deren Herstellung und Verwendung zur selektiven Hydrierung von ungesättigten Carbonylverbindungen. Der verwendete Ru/Fe/C-Katalysa-tor wird durch Dotierung von Aktivkohle mit Rutheniumchloridhydrat, Trocknen und Vermischen mit Eisenoxidpulver und Reduktion bei 500°C hergestellt. Bei der katalytischen Reaktion werden Methanol und Trimethylamin zur Selektivitätssteigerung zugegeben. Die Selektivitäten betragen 2,3 % für Citronellol und 96,5 % für Geraniol/Nerol bei 100 % Umsatz. Durch den Zusatz von Methanol und Trimethylamin ist jedoch eine zusätzliche Trennstufe bei der Aufarbeitung des Reaktionsaustrages erforderlich.

EP 422 968 beschreibt die Hydrierung von Citral mit einem SiO₂-Trägerkatalysator, der als Aktivkomponente 0,1-10 Gew.-% Ir, Pt, Rh oder Ru enthält und mit 0,01 bis 10 Gew.-% Zinn, Blei oder Germanium dotiert ist. Die Reaktion wird in Hexan durchgeführt. Die erhaltenen Citronellol-Selektivitäten sind größer 3,5 %.

Bei den im Stand der Technik beschriebenen Verfahren zur Hydrierung von α,β-ungesättigten Carbonylverbindungen ist die Selektivität zu den ungesättigten Alkoholen nicht befriedigend. Insbesondere bei der Hydrierung von Citral liegt die Selektivität zu Citronellol bei hohen Citral-Umsätzen bei über 2 %. Da sich Citronellol/Nerol-Gemische destillativ sehr schlecht trennen lassen, schränkt dies die industrielle Verwertbarkeit wesentlich ein. Ein vorteilhafter Katalysator sollte auch bei Citral-Umsätzen größer als 95 % Citronellol-Selektivitäten unterhalb von 2 Gew.-% ermöglichen.

Bei den vorbeschriebenen Verfahren wird häufig mit Lösungsmitteln, teilweise auch unter Zusatz von Hilfsstoffen wie Trimethylamin gearbeitet. Dies erhöht den Aufwand der destillativen Aufarbeitung. Weiterhin wird das Reaktorvolumen vergrößert, was die Wirtschaftlichkeit des Verfahrens erheblich verschlechtert.

Aufgabe der Erfindung war es, ein verbessertes Verfahren zur Hydrierung von α,β- ungesättigten Carbonylverbindungen zu den entsprechenden Alkohlen, insbesondere zur Hydrierung von Citral zu Geraniol/Nerol, zu entwickeln, das bei hohen Citralumsätzen auf wirtschaftlichere Weise zu niedrigen Citronellol-Selektivitäten führt.

Erfindungsgemäß gelöst wurde die Aufgabe durch ein Verfahren zur selektiven Flüssigphasenhydrierung von α,β- ungesättigten Carbonylverbindungen der allgemeinen Formel I, 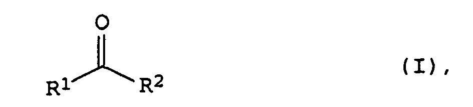 wobei
- R¹, R²: jeweils unabhängig voneinander gleich oder verschieden sein können und Wasserstoff oder einen gesättigten oder einen ein- oder mehrfach ungesättigten geradkettigen oder verzweigten gegebenenfalls substituierten C₁-C₂₀-Alkylrest, einen gegebenenfalls substituierten Arylrest oder eine gegebenenfalls substituierte heterocyclische Gruppe darstellen,
zu den entsprechenden Alkoholen der allgemeinen Formel II 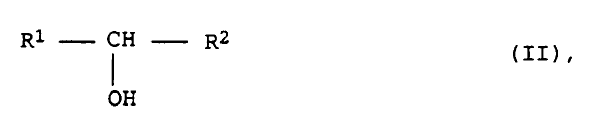 wobei R¹ und R² die oben angegebene Bedeutung haben
in Gegenwart von Wasserstoff und einem Pt/ZnO-Katalysator.

Der Katalysator hydriert mit überraschend hoher Selektivität die Aldehyd-Gruppe der ungesättigten Carbonylverbindung.

Unter einem ein oder mehrfach ungesättigten geradkettigen oder verzweigten C₁-C₂₀-Alkylrest versteht man, wenn nicht anders angegeben einen Methyl, Ethyl, Propyl, Isopropyl, n-Butyl, i-Butyl-, t-Butyl, Pentyl, Hexyl, Heptenyl, Octyl-, Nonyl-, Decyl, 1-Propenyl-, 2-Propenyl-, 2-Methyl-2-propenyl, 1-Pentenyl, 1-Methyl-2-Pentenyl-, Isopropenyl-, 1-Butenyl-, Hexenyl-, Heptenyl-, Octenyl-, Nonenyl-, oder einen Decenylrest bzw. die den weiter unten aufgeführten eingesetzten Verbindungen entsprechenden Reste.

Unter einem C₁-C₄-Alkylrest versteht man, wenn nicht anders angegeben einen Methyl-, Ethyl-, Propyl-, i-Propyl-, Butyl- oder t-Butylrest.

Unter einem Arylrest versteht man einen Benzyl, Phenyl- oder Naphthylrest.

Unter einer heterocyclischen Gruppe versteht man beispielsweise einen Pyridin-, Pyrimidin, Pyridazin, Pyrazin-, Piperazin-, Imidazol-, Furan, Oxazol, Isothiazol, Isoxazol, 1,2,3-Triazol- oder 1,2,4-Triazol, Thiazol-, Thiophen- oder Indolring.

Substituenten können Methyl-, Ethyl-, Propyl-, i-Propyl-, Butyl-, t-Butyl-, Fluor-, Chlor-, Brom-, Jod-, Nitro- oder Aminoreste bedeuten.

Als α,β-ungesättigte Carbonylverbindungen können beispielsweise Acrolein, Methacrolein, Crotonaldehyd, Prenal, Farnesal oder Citral, insbesonders bevorzugt Citral eingesetzt werden.

In einer bevorzugten Ausgestaltung des Verfahrens wird Citral zu Geraniol bzw. Nerol umgesetzt.

Der verwendete Pt/ZnO-Katalysator kann sowohl als Vollkatalysator als auch, um die mechanische Stabilität zu erhöhen, als Trägerkatalysator eingesetzt werden. Als Trägermaterialien können alle üblichen Trägermaterialien, z. B. γ-Al₂O₃, α-Al₂O₃, SiO₂, Aktivkohle, TiO₂, ZrO₂, Zeolithe oder monolithische Packungsstrukturen eingesetzt werden.

Bevorzugt wird der Katalysator aber als Vollkatalysator eingesetzt.

Das Verfahren kann sowohl kontinuierlich als auch diskontinuierlich in Suspension oder im Festbett durchgeführt werden. Besonders vorteilhaft ist die kontinuierliche Fahrweise.

Für die Suspensions- oder Festbettvariante bieten sich übliche Reaktorkonzepte an, wie sie z.B. in Ullmann's Encyclopedia of Industrial Chemistry, Sixth Edition, 2000 Electronic Release, beschrieben sind.

Das kontinuierliche bzw. diskontinuierliche Suspensionsverfahren kann beispielsweise wie in EP 947 493 bzw. US 5,939,589 beschrieben durchgeführt werden. Der Katalysator wird sowohl in der diskontinuierlichen als auch in der kontinuierlichen Suspensionfahrweise in feinverteilter Form eingesetzt, wobei die Teilchengröße kleiner 1 mm, bevorzugt zwischen 1 und 100 µm ist.

Für die Festbettvariante wird der Katalysator in Strang- oder Splitform eingesetzt. Typische Strangdurchmesser liegen zwischen 1 und 5 mm, die Stranglängen liegen bei 1 bis 20 mm. Der Reaktor kann in Riesel- oder Sumpffahrweise betrieben werden.

Die Reaktion wird sowohl bei der Suspensionsfahrweise als auch bei der Festbettvariante drucklos oder unter einem Druck von 1 bis 200 bar, bevorzugt 10 bis 100 bar, insbesonders bevorzugt bei 20 bis 50 bar durchgeführt. Die Temperaturen liegen zwischen 25 und 200°C, bevorzugt zwischen 80 und 150°C. Die Reaktion kann sowohl mit als auch ohne Lösungsmittel durchgeführt werden. Als Lösungsmittel können niedere Alkohole wie Methanol, Ethanol oder Isopropylalkohol eingesetzt werden. Weiterhin kann bei Bedarf eine organische Base, wie Trimethylamin zugesetzt werden. Bevorzugt wird das Verfahren aber ohne die Verwendung eines Lösungsmittels und ohne die Verwendung einer zusätzlichen Base durchgeführt.

Der verwendete Pt/ZnO-Katalysator enthält 0,1-10 Gew.-% Pt, bevorzugt 2 - 8 Gew-%, die BET-Oberfläche liegt zwischen 1 und 30 m²/g⁻¹ und die Pt-Partikelgröße liegt bei 1 bis 10 nm. Die Platinpartikel lagern sich teilweise zu Agglomeraten mit einer Größe von 10 bis 100 nm zusammen.

Die nachfolgenden Beispiele sollen die Erfindung verdeutlichen, ohne sie jedoch darauf zu beschränken:

### Beispiele

### Katalysatorherstellung:

### Vollkatalysator

200g ZnO Stränge von 4 mm Durchmesser wurden mit einer wässrigen Lösung von Hexachloroplatinsäure [H₂PtCl₆xH₂O] getränkt. Hierzu wurden 20,9 g der Hexachloroplatinsäure in 56 g destilliertem Wasser gelöst. Die feuchten Stränge wurden bei 120°C getrocknet und dann für 4 h bei 400°C an Luft getempert. Anschließend wurde der Katalysator in eine Reduktionskolonne eingebaut und für 2 h bei 200°C reduziert. Vor dem Ausbau aus der Reduktionskolonne wurde der Katalysator bei Raumtemperatur in einem verdünnten Luftstrom passiviert. Vor den Autoklaventests wurde der Katalysator auf eine Partikelgröße < 100 µm zerkleinert.

Der so hergestellte Katalysator hatte einen Pt-Gehalt von 5,3 Gew.-% und einen Chlor-Gehalt von 1,5 Gew.-%.

### Trägerkatalysator

Zur Herstellung eines Trägerkatalysators wird der Träger zunächst mit Zn-Salzen wie Zn(NO₃)₂ oder ZnCl₂ getränkt und durch anschließendes Trockenen bei 120 °C (50-200 °C), 1-6 h, und Kalzinieren bei 200-500 °C (1-6 h) wird das Zn-Salz in ZnO überführt, das dann fest auf dem Träger liegt. Anschließend kann wie bei dem ungeträgerten Katalysator das Pt als H₂PtCl₆ aufgetränkt, getrocknet, kalziniert und reduziert werden. Der ZnO-Gehalt des Katalysators liegt zwischen 1 und 90 Gew.-%, der Pt-Gehalt zwischen 0,1 und 10 Gew.-%.

### Katalysatortestung

### Beispiel 1

5 g des Katalysatorpulvers, das aus dem Vollkatalysator hergestellt wurde, wurden in einen Autoklaven von 300 ml Volumen gegeben. Hierzu wurden 250 ml Citral, d.h. eine Mischung aus etwa 50 % Geranial und 50 % Neral, gegeben. Nach Verschließen des Autoklaven wurde unter Rühren im Stickstoffstrom auf 140°C aufgeheizt. Nach Erreichen der Endtemperatur wurde der Stickstoff durch Wasserstoff ersetzt und auf einen Druck von 50 bar aufgepresst. Während der Reaktion wurden 50 l/h Abgas gefahren.

Bei diesem Test wurden in Abhängigkeit der Reaktionsdauer die in Tabelle 1 angegebenen Ergebnisse erhalten.

**Tabelle 1**

| | | | | | | | | |
|---|---|---|---|---|---|---|---|---|
| **Reaktionsdauer/min** | **120** | **240** | **360** | **480** | **600** | **720** | **840** | **900** |
| Umsatz Citral % | 10,5 | 24,0 | 45,7 | 70,5 | 90,7 | 99,0 | 99,3 | 99,4 |
| **Selektivitäten %** | | | | | | | | |
| Citronellal | 1,9 | 1,3 | 0,8 | 0,4 | 0,2 | 0,0 | 0,0 | 0,0 |
| Citronellol | 0,4 | 0,4 | 0,6 | 0,9 | 1,1 | 1,4 | 1,5 | 1,5 |
| Nerol | 20,6 | 29,8 | 32,7 | 33,6 | 34,1 | 35,4 | 35,5 | 35,1 |
| Geraniol | 27,5 | 43,4 | 50,4 | 52,4 | 52,7 | 53,0 | 52,8 | 52,2 |
| Unbekannte | 20,4 | 12,9 | 9,6 | 9,5 | 9,3 | 8,1 | 8,2 | 9,1 |

### Beispiel 2 (Festbett)

In eine kontinuierlich betriebene Laborapparatur (Reaktorgeometrie: Länge 171 mm /Durchmesser: 27,3 mm 100 ml Reaktorinhalt, betrieben im geraden Durchgang) wurde der strangförmige Vollkatalysator eingebaut und der Reaktor mit Wasserstoff (20 Nl/h) gespült. Anschließend wurde der Reaktor mit Wasserstoff auf 20 bar aufgepresst. Die Temperatur wurde dann auf Reaktionstemperatur gebracht und der Druck auf 40 bar erhöht. Das Edukt wurde dann bei verschiedenen Temperaturen (60-150°C, und unterschiedlichen Belastungen 0,2-0,6 kg/l*h) in Sumpffahrweise über den Katalysator gefahren. Das Produkt wurde in einen Vorratsbehälter entspannt und mittels Gaschromatographie (50m DB1 0,24mm 0,4 micrometer, 130°C - 1°C/min- 155°C - 20°C/min- 280°C) analysiert.

Dabei wurden die in Tabelle 2 aufgeführten Ergebnisse erhalten:

**Tabelle 2**

| | | | Selektivitäten | | |
|---|---|---|---|---|---|
| T/°C | Belastung kg/l/h | Umsatz | Citronellol | Nerol | Geraniol |
| 100 | 0,4 | 70 | 0,58 | 43 | 49 |
| 120 | 0,4 | 93 | 1,1 | 45 | 46 |

### Beispiel 3

Es wurde eine Kreislaufapparatur in Sumpffahrweise gewählt. Nachdem der reduziert-passivierte Katalysator in den Rohrreaktor eingebaut worden war, wurde die Apparatur mit 0,55 Liter Citral befüllt. Nach dem Aufpressen von Wasserstoff auf 50 bar wurde die Kreislaufpumpe in Betrieb genommen und eine Querschnittsbelastung von 100 m³/m²/h bezogen auf den freien Reaktorquerschnitt eingestellt. Mittels eines Vorheizers wurde die Reaktoreintrittstemperatur auf 120°C gebracht. Während der Hydrierung wurden in bestimmten Zeitabständen Proben aus dem Kreislauf entnommen und gaschromatographisch analysiert. Ein Abgasstrom von 50 L/h wurde permanent eingestellt, um den CO Gehalt unter 10 ppm während der gesamten Betriebszeit einzuhalten.

| Citral | Nerol | Geraniol | Citronellal | Isopulegol | Citronellol | NP ab RT 43,5 | Umsatz | Selektivität | Ausbeute | CO im Abgas |
|---|---|---|---|---|---|---|---|---|---|---|
| | | | | | | | | | | |
| 96,33 | 0,12 | 0,13 | 0,03 | 1,77 | 0,01 | <0,5 | 0 | 0 | 0 | 0 |
| 91,93 | 2,18 | 2,60 | 0,16 | 1,34 | 0,05 | <0,5 | 4,57 | 100,00 | 4,57 | 16 |
| 82,48 | 6,27 | 7,30 | 0,25 | 1,47 | 0,11 | <0,5 | 14,38 | 97,98 | 14,09 | 11 |
| 73,53 | 10,37 | 11,92 | 0,32 | 1,43 | 0,19 | <0,5 | 23,67 | 97,76 | 23,14 | 9 |
| 65,21 | 14,11 | 15,94 | 0,38 | 1,49 | 0,27 | <0,5 | 32,31 | 96,56 | 31,19 | 8 |
| 50,12 | 21,43 | 23,50 | 0,45 | 1,26 | 0,48 | <0,5 | 47,97 | 97,23 | 46,64 | 8 |
| 45,51 | 23,57 | 25,36 | 0,49 | 1,36 | 0,58 | 0,51 | 52,76 | 96,28 | 50,79 | 7,0 |
| 39,93 | 26,32 | 28,11 | 0,48 | 1,17 | 0,69 | 0,61 | 58,55 | 96,51 | 56,50 | 8,5 |
| 18,13 | 37,71 | 38,25 | 0,39 | 0,70 | 1,22 | 0,77 | 81,18 | 97,14 | 78,85 | 9 |
| 6,70 | 44,32 | 43,10 | 0,21 | 0,37 | 1,65 | 0,74 | 93,04 | 97,53 | 90,75 | 9 |
| 2,07 | 47,26 | 44,86 | 0,11 | 0,21 | 1,92 | 0,65 | 97,85 | 97,73 | 95,63 | 8 |
| 0,65 | 48,25 | 45,34 | 0,30 | 0,14 | 2,07 | 0,57 | 99,33 | 97,82 | 97,16 | 8 |
| 0,30 | 48,54 | 45,52 | 0,04 | 0,13 | 2,16 | 0,51 | 99,69 | 97,95 | 97,64 | 7 |

| | | | | | | | | | | |
|---|---|---|---|---|---|---|---|---|---|---|
| Angaben in GC-Fl.-%, CO-Gehalt in ppm | | | | | | | | | | |

Aus dieser Tabelle geht hervor, daß mit dem erfindungsgemäßen Katalysator selbst bei hohen Citral-Umsätzen >99 % insgesamt eine Geraniol/Nerol-Ausbeute von > 97% mit nur 2 % Citronellol erreicht worden ist.

### Vergleichsbeispiele zu Beispiel 3

| Versuch | Katalysator | T | P | Citral cis/ trans | Nerol | Geranio 1 | Citronellal | Citronel lol | Umsatz | Selektivi tät |
|---|---|---|---|---|---|---|---|---|---|---|
| Nr. | | °C | Bar | Fl-% | Fl-% | Fl-% | Fl-% | Fl-% | % | % |
| 1 | Ru / Al₂O₃ | 100 | 50 | 19,67 | 21,51 | 30,40 | 5,96 | 18,76 | 79,55 | 67,84 |
| 2 | Pt / Al₂O₃ | 100 | " | 82,09 | 1,98 | 2,67 | 1,23 | 4,77 | 4,93 | 26,29 |
| 3 | Cu/Cr Al₂O₃ | 175 | " | 37,57 | 12,34 | 18,29 | 15,62 | 7,16 | 61,45 | 51,14 |

## Patentansprüche

1. Verfahren zur selektiven Flüssigphasenhydrierung von α,β-ungesättigten Carbonylverbindungen der allgemeinen Formel I, 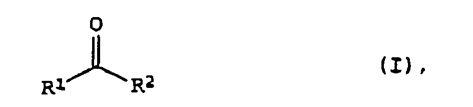 wobei
R¹, R² jeweils unabhängig voneinander gleich oder verschieden sein können und Wasserstoff oder einen gesättigten oder einen ein- oder mehrfach ungesättigten geradkettigen oder verzweigten gegebenenfalls substituierten C₁-C₂₀-Alkylrest, einen gegebenenfalls substituierten Arylrest oder eine gegebenenfalls substituierte heterocyclische Gruppe darstellen,
zu den entsprechenden Alkoholen der allgemeinen Formel II 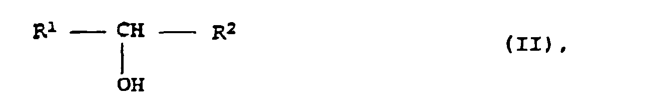 wobei R¹ und R² die oben angegebene Bedeutung haben
in Gegenwart von Wasserstoff und einem Pt/ZnO-Katalysator.

2. Verfahren gemäß Anspruch 1, **dadurch gekennzeichnet, dass** die Carbonylverbindung Citral ist.

3. Verfahren gemäß einem der Ansprüche 1 bis 2, **dadurch gekennzeichnet, dass** es in kontinuierlicher Fahrweise durchgeführt wird.

4. Verfahren gemäß einem der Ansprüche 1 bis 3, **dadurch gekennzeichnet, dass** der Pt/ZnO-Katalysator auf einem Träger oder als Vollkatalysator eingesetzt wird.

5. Verfahren gemäß einem der Ansprüche 1 bis 4, **dadurch gekennzeichnet, dass** das Verfahren in Suspension durchgeführt wird.

6. Verfahren gemäß einem der Ansprüche 1 bis 5, **dadurch gekennzeichnet, dass** die Teilchengröße des Katalysators kleiner 1 mm ist.

7. Verfahren gemäß einem der Ansprüche 1 bis 6, **dadurch gekennzeichnet, dass** das Verfahren im Festbett durchgeführt wird.

8. Verfahren gemäß Anspruch 7, **dadurch gekennzeichnet, dass** der Katalysator in Strangform eingesetzt wird.

9. Verfahren gemäß einem der Ansprüche 1 bis 8, **dadurch gekennzeichnet, dass** das Verfahren drucklos oder bei einem Druck von 10 bis 100 bar durchgeführt wird.

10. Verfahren gemäß einem der Ansprüche 1 bis 9, **dadurch gekennzeichnet, dass** das Verfahren bei Temperaturen zwischen 25 und 200°C durchgeführt wird.

11. Verfahren gemäß einem der Ansprüche 1 bis 10, **dadurch gekennzeichnet, dass** der Pt/ZnO-Katalysator 0,1 bis 10 Gew.-% Pt enthält.

12. Verfahren gemäß einem der Ansprüche 1 bis 11, **dadurch gekennzeichnet, dass** die Pt-Partikelgröße des verwendeten Katalysators zwischen 1 und 10 nm liegt.

13. Verfahren gemäß einem der Ansprüche 1 bis 12, **dadurch gekennzeichnet, dass** der CO-Gehalt durch das Einstellen eines Abgasstromes unterhalb von 100 ppm gehalten wird.

14. Verfahren gemäß einem der Ansprüche 1 bis 13, **dadurch gekennzeichnet, dass** der CO-Gehalt durch das Einstellen eines Abgasstromes unterhalb von 10 ppm gehalten wird.

15. Verwendung eines Pt/ZnO-Katalysators zur selektiven Flüssigphasenhydrierung von α,β-ungesättigten Carbonylverbindungen der allgemeinen Formel I, 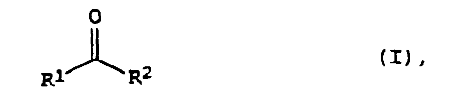 wobei
R¹, R² jeweils unabhängig voneinander gleich oder verschieden sein können und Wasserstoff oder einen gesättigten oder einen ein- oder mehrfach ungesättigten geradkettigen oder verzweigten gegebenenfalls substituierten C₁-C₂₀-Alkylrest, einen gegebenenfalls substituierten Arylrest oder eine gegebenenfalls substituierte heterocyclische Gruppe darstellen,
zu den entsprechenden Alkoholen der allgemeinen Formel II 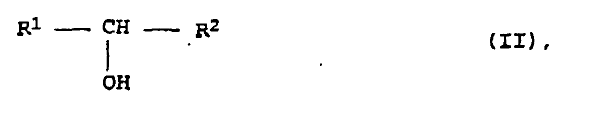 wobei R¹ und R² die oben angegebene Bedeutung haben
in Gegenwart von Wasserstoff und einem Pt/ZnO-Katalysator.

## Claims

1. The process for selective liquid phase hydrogenation of α,β-unsaturated carbonyl compounds of the general formula I 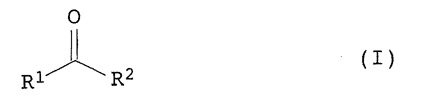 where
R¹ and R² are identical or different and are each independently hydrogen or a saturated or a mono- or polyunsaturated straight-chain or branched, optionally substituted C₁-C₂₀-alkyl radical, an optionally substituted aryl radical or an optionally substituted heterocyclic group,
to give the corresponding alcohols of the general formula II 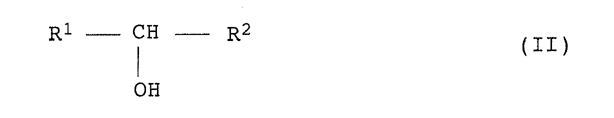
where R¹ and R² are each as defined above,
in the presence of hydrogen and a Pt/ZnO catalyst.

2. The process according to claim 1, wherein the carbonyl compound is citral.

3. The process according to any of claims 1 to 2 that is carried out in a continuous manner.

4. The process according to any of claims 1 to 3, wherein the Pt/ZnO catalyst is used in a supported or unsupported form.

5. The process according to any of claims 1 to 4, wherein the process is carried out in suspension.

6. The process according to any of claims 1 to 5, wherein the particle size of the catalyst is smaller than 1 mm.

7. The process according to any of claims 1 to 6, wherein the process is carried out in a fixed bed.

8. The process according to claim 7, wherein the catalyst is used in extruded form.

9. The process according to any of claims 1 to 8, wherein the process is carried out at atmospheric or at a pressure of from 10 to 100 bar.

10. The process according to any of claims 1 to 9, wherein the process is carried out at from 25 to 200°C.

11. The process according to any of claims 1 to 10, wherein the Pt/ZnO catalyst comprises from 0.1 to 10% by weight of Pt.

12. The process according to any of claims 1 to 11, wherein the Pt particle size of the catalyst employed is in the range from 1 to 10 nm.

13. The process according to any of claims 1 to 12, wherein the CO content is held below 100 ppm by adjusting an off-gas stream.

14. The process according to any of claims 1 to 13, wherein the CO content is held below 10 ppm by adjusting an off-gas stream.

15. The use of a Pt/ZnO catalyst for the selective liquid phase hydrogenation of α,β-unsaturated carbonyl compounds of general formula I, 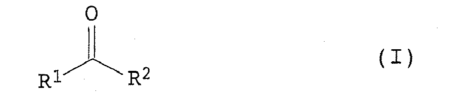 where
R¹ and R² are identical or different and are each independently hydrogen or a saturated or a mono- or polyunsaturated straight-chain or branched, optionally substituted C₁-C₂₀-alkyl radical, an optionally substituted aryl radical or an optionally substituted heterocyclic group,
to give the corresponding alcohols of the general formula II 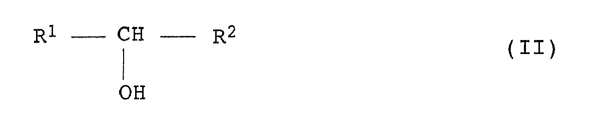
where R¹ and R² are each as defined above
in the presence of hydrogen and a Pt/ZnO catalyst.

## Revendications

1. Procédé d'hydrogénation sélective en phase liquide de composés carbonyle α,β-insaturés de la formule générale I 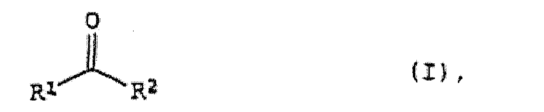 dans laquelle
R¹, R² peuvent chacun indépendamment l'un de l'autre être identiques ou différents et représentent de l'hydrogène ou un radical alkyle en C₁-C₂₀ saturé ou insaturé à une ou à plusieurs reprises, linéaire ou ramifié, éventuellement substitué, un radical aryle éventuellement substitué ou un groupe hétérocyclique éventuellement substitué,
en les alcools correspondants de la formule générale II 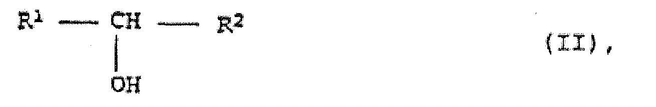 dans laquelle R¹ et R² ont la signification donnée ci-dessus,
en présence d'hydrogène et d'un catalyseur Pt/ZnO.

2. Procédé suivant la revendication 1, **caractérisé en ce que** le composé carbonyle est du citral.

3. Procédé suivant l'une des revendications 1 et 2, **caractérisé en ce qu'**il est effectué selon un mode de conduite continu.

4. Procédé suivant l'une des revendications 1 à 3, **caractérisé en ce que** le catalyseur Pt/ZnO est mis en oeuvre sur un support ou sous la forme d'un catalyseur massique.

5. Procédé suivant l'une des revendications 1 à 4, **caractérisé en ce que** le procédé est effectué en suspension.

6. Procédé suivant l'une des revendications 1 à 5, **caractérisé en ce que** la taille des particules du catalyseur est inférieure à 1 mm.

7. Procédé suivant l'une des revendications 1 à 6, **caractérisé en ce que** le procédé est effectué dans un lit fixe.

8. Procédé suivant la revendication 7, **caractérisé en ce que** le catalyseur est mis en oeuvre sous la forme d'extrudé.

9. Procédé suivant l'une des revendications 1 à 8, **caractérisé en ce que** le procédé est effectué sans pression ou à une pression de 10 à 100 bars.

10. Procédé suivant l'une des revendications 1 à 9, **caractérisé en ce que** le procédé est effectué à des températures comprises entre 25 et 200°C.

11. Procédé suivant l'une des revendications 1 à 10, **caractérisé en ce que** le catalyseur à base de Pt/ZnO contient 0,1 à 10% en poids de Pt.

12. Procédé suivant l'une des revendications 1 à 11, **caractérisé en ce que** la taille des particules de Pt du catalyseur utilisé est située entre 1 et 10 nm.

13. Procédé suivant l'une des revendications 1 à 12, **caractérisé en ce que** la teneur en CO est, par l'ajustement d'un courant de gaz résiduaire, maintenue en dessous de 100 ppm.

14. Procédé suivant l'une des revendications 1 à 13, **caractérisé en ce que** la teneur en CO est, par l'ajustement d'un courant de gaz résiduaire, maintenue en dessous de 10 ppm.

15. Utilisation d'un catalyseur à base de Pt/ZnO pour l'hydrogénation sélective en phase liquide de composés carbonyle α,β-insaturés de la formule générale I 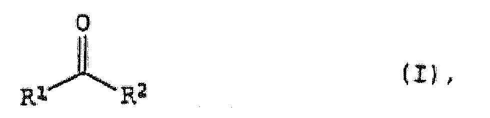 dans laquelle
R¹, R² peuvent être chacun indépendamment l'un de l'autre identiques ou différents et représentent de l'hydrogène ou un radical alkyle en C₁-C₂₀ saturé ou insaturé à une ou à plusieurs reprises, linéaire ou ramifié, éventuellement substitué, un radical aryle éventuellement substitué ou un groupe hétérocyclique éventuellement substitué,
en les alcools correspondants de la formule générale II 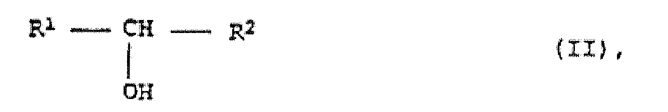 dans laquelle R¹ et R² ont la signification indiquée ci-dessus,
en présence d'hydrogène et d'un catalyseur à base de Pt/ZnO.
